(19) Europäisches Patentamt
European Patent Office
Office européen des brevets

(11) **EP 2 892 427 B1**

(12) **EUROPÄISCHE PATENTSCHRIFT**

(45) Veröffentlichungstag und Bekanntmachung des Hinweises auf die Patenterteilung:
**03.06.2020 Patentblatt 2020/23**

(21) Anmeldenummer: **13745100.1**

(22) Anmeldetag: **05.08.2013**

(51) Int Cl.:
**A61B 5/08** *(2006.01)*  **A61M 16/00** *(2006.01)*
**A61B 5/085** *(2006.01)*

(86) Internationale Anmeldenummer:
**PCT/EP2013/066362**

(87) Internationale Veröffentlichungsnummer:
**WO 2014/037175 (13.03.2014 Gazette 2014/11)**

(54) **SYSTEM ZUR AUTOMATISIERTEN EINSTELLUNG EINES DURCH EINE BEATMUNGSEINRICHTUNG VORGEGEBENEN DRUCKS**

SYSTEM FOR AUTOMATIC ADJUSTMENT OF A PRESSURE SPECIFIED BY A RESPIRATOR DEVICE

SYSTÈME D'AJUSTEMENT AUTOMATIQUE D'UNE PRESSION PRESCRITE PAR UN DISPOSITIF RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.09.2012 DE 102012215662**

(43) Veröffentlichungstag der Anmeldung:
**15.07.2015 Patentblatt 2015/29**

(73) Patentinhaber: **Hamilton Medical AG**
**7402 Bonaduz (CH)**

(72) Erfinder:
• **NOVOTNI, Dominik**
 **7000 Chur (CH)**
• **LAUBSCHER, Thomas**
 **7403 Rhäzüns (CH)**

(74) Vertreter: **Schmitt-Nilson Schraud Waibel Wohlfrom**
**Patentanwälte Partnerschaft mbB**
**Pelkovenstraße 143**
**80992 München (DE)**

(56) Entgegenhaltungen:
**EP-A1- 2 397 074  WO-A1-2007/082384**
**DE-A1-102009 023 965**

• **TALMOR DANIEL ET AL: "Mechanical Ventilation Guided by Esophageal Pressure in Acute Lung Injury", NEW ENGLAND JOURNAL OF MEDICINE, THE - NEJM -, MASSACHUSETTS MEDICAL SOCIETY, US, vol. 359, no. 20, 13 November 2008 (2008-11-13), pages 2095-2104, XP002607678, ISSN: 0028-4793, DOI: 10.1056/NEJMOA0708638 [retrieved on 2010-11-01]**

EP 2 892 427 B1

**Beschreibung**

Die vorliegende Erfindung betrifft ein System zur automatisierten Einstellung eines durch eine Beatmungseinrichtung vorgegebenen Drucks, insbesondere eines positiven endexpiratorischen Drucks und/oder eines maximalen Atemwegsdrucks. Die Erfindung betrifft auch eine Einrichtung zur maschinellen Beatmung, die über ein solches System zur Einstellung des vorgegebenen Drucks, insbesondere des positiven endexpiratorischen Drucks und/oder des maximalen Atemwegsdrucks, verfügt.

Bei heute gängigen Formen der maschinellen Beatmung wird dem Patienten Atemgas mit Überdruck zugeführt. Deswegen ist bei der Beatmung der Atemwegsdruck bzw. alveoläre Druck zumindest während der Inspirationsphase größer als der Druck im die Lungenbläschen bzw. Alveolen umgebenden Pleuraspalt. Während der Expirationsphase erfolgt keine Druckbeaufschlagung des Atemwegs durch die Beatmungseinrichtung, mit der Folge, dass das Lungengewebe sich entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Expirationsphase derart ungünstig einstellen, dass es zu einem Kollaps von Teilen der Alveolen kommt. Dann muss der kollabierte Teil des Lungenvolumens im nachfolgenden Atemzyklus erst wieder von Neuem entfaltet werden. Die funktionelle Residualkapazität der Lunge wird stark beeinträchtigt, so dass die Sauerstoffsättigung abnimmt, und auch das Lungengewebe nimmt dauerhaft Schaden.

Um einen Kollaps von Alveolen am Ende der Expirationsphase zu verhindern, wird bei der maschinellen Überdruckbeatmung in der Regel ein sog. positiver endexpiratorischer Druck, in der Regel kurz als PEEP bezeichnet, eingestellt. Mit dieser Maßnahme kann in vielen Fällen eine Verbesserung der Sauerstoffsättigung erreicht werden.

Bei Beatmung mit PEEP beaufschlagt die Beatmungseinrichtung den Atemweg dauerhaft - also sowohl während der Inspirationsphase als auch während der Expirationsphase - mit einem vorbestimmten Überdruck, dem PEEP. Der PEEP liegt also auch nach dem Ende der Expirationsphase noch an.

Idealerweise sollte der PEEP genügend groß eingestellt werden, so dass während der Expirationsphase der alveoläre Druck nicht, oder jedenfalls nur so weit, unterhalb dem Druck im Pleuraspalt liegt, dass das alveoläre Gewebe unter der Wirkung des Drucks im Pleuraspalt nicht kollabiert. Mit anderen Worten: Der PEEP soll verhindern, dass der transpulmonale Druck - das ist die Druckdifferenz zwischen alveolärem Druck und Druck im Pleuraspalt - kleiner wird als Null bzw. einen unteren negativen Grenzwert unterschreitet, ab dem Teile der Alveolen zu kollabieren beginnen.

Andererseits kann sich ein zu hoher Wert des PEEP negativ auswirken, insbesondere während der Inspirationsphase. Denn das Lungengewebe kann bei sehr hohen Atemwegsdrücken während der Inspirationsphase überdehnt werden. Zahlreiche Studien deuten außerdem darauf hin, dass ein hoher Wert des PEEP den Rückfluss von venösem Blut zum Herzen behindern kann mit entsprechend negativen Auswirkungen auf das Herz-Kreislaufsystem.

In der klinischen Praxis wird der PEEP von Ärzten bzw. Pflegepersonal von Intensivstationen aufgrund vorgegebener physiologischer Parameter eines Patienten bzw. anhand von bekannten therapeutischen Richtwerten wie den sogenannten "ARDSnet-Richtlinien" eingestellt, siehe beispielsweise The Acute Respiratory Distress Syndrome Network, The New England Journal of Medicine, 2000, 342: S. 1301-1308 oder Grasso et al., American Journal of Respiratory Critical Care, 2007, 176: S. 761-767. Eine solche Einstellung erfolgt in der Regel im Vorhinein und wird nur sporadisch durch Ärzte oder Pflegepersonal angepasst.

An sich sollte der PEEP an den jeweils herrschenden transpulmonalen Druck angepasst sein. Der transpulmonale Druck bei einem beatmeten Patienten ist aber einer einfachen Bestimmung nicht zugänglich. In der Praxis muss man sich mit Abschätzungen des transpulmonalen Drucks auf Grundlage früherer klinischer Studien, wie den bereits angesprochenen ARDSnet-Richtlinien, behelfen. Solche Richtlinien können naturgemäß nicht den tatsächlichen Zustand eines Patienten reflektieren, sondern geben Erfahrungswerte wieder. Beispielsweise wird vermutet, dass bei Patienten mit verhältnismäßig steifer Brust ein nach Maßgabe der ARDSnet-Richtlinien eingestellter PEEP häufig zu niedrig sein wird, mit der Folge, dass der beatmete Patient nicht ausreichend oxygeniert wird, selbst dann nicht, wenn eine Einstellung des PEEP nach Maßgabe der vorgegebenen Richtlinien erfolgt.

Ein großer Nachteil bekannter Verfahren zur Ableitung eines für einen jeweiligen Patienten optimalen PEEP liegt darin, dass die erforderlichen Messprozeduren eine beträchtliche Zeit in Anspruch nehmen, während der eine Beatmung des Patienten in einem regelmäßigen Atemzyklus nicht möglich ist. Dies ist etwa bei mit Hilfe der "Super-Syringe-Methode" aufgenommenen statischen Druck/Volumen Kurven (P/V-Kurven) der Fall, wie sie beispielsweise von Brochard L., Critical Care, 2006, 10: p. 156-158 beschrieben werden.

Aus der EP 2 091 429 B1 ist ein System zur automatischen Einstellung des PEEP bekannt, welches in bestimmten Zeitabständen ein P/V-Manöver durchführt, bei dem der Beatmungsdruck in einem vorgegebenen zeitlichen Muster erhöht und verringert wird und die sich dabei ergebende Änderung des Lungenvolumens erfasst wird. Der PEEP wird dann anhand von während dieses Manövers erfassten P/V-Kurven bestimmt, und zwar anhand desjenigen Drucks, bei dem eine Volumendifferenz zwischen der P/V Kurve beim Einatmen und der P/V Kurve beim Ausatmen maximal wird. Auch ein derartiges P/V Manöver lässt sich nur sporadisch und unter Aufsicht durchführen. Die Möglichkeit der automatischen Einstellung des PEEP durch die Beatmungseinrichtung ist damit zwar grundsätzlich gegeben, kann jedoch in der Praxis nicht zufriedenstellend genutzt werden.

TALMOR DANIEL ET AL: "Mechanical Ventilation Guided by Esophageal Pressure in Acute Lung Injury", NEW ENGLAND JOURNAL OF MEDICINE, THE - NEJM -, MASSACHUSETTS MEDICAL SOCIETY, US, Bd. 359, Nr. 20, 13. November 2008 (2008-11-13), Seiten 2095-2104, CP0026607678, ISSN: 0028-4793, DOI: 10.1056/NEJMOA0708638 zeigt einen Vergleich von zwei Verfahren zum mechanischen Beatmen von Patienten mit akuten Lungenverletzungen. Das erste Verfahren, wird nach den sogenannten "ARDSnet-Richtlinien" durchgeführt und verwendet als Einstellparameter ein Atemwegvolumen von 6ml pro Kilogramm Körpergewicht und einen PEEP Wert basierend auf PaO2 und FIO2-Pati-entenwerten. Das zweite Verfahren verwendet als Einstellparameter einen Speiseröhrendruck, der mit Hilfe eines Speiseröhrenballonkatheters ermittelt wird.

Der vorliegenden Erfindung liegt die Aufgabe zugrunde, ein System anzugeben, das die Möglichkeit der ganz oder jedenfalls weitgehend automatischen patientenbezogenen Einstellung bzw. Nachstellung eines durch die Beatmungseinrichtung vorgegebenen Drucks ermöglicht. Der durch die Beatmungseinrichtung vorgegebene Druck kann beispielsweise der positive endexpiratorische Druck PEEP sein. Zusätzlich oder alternativ soll es möglich sein, den maximalen Atemwegsdruck oder einen anderen von der Beatmungseinrichtung vorgegebenen Druck ganz oder jedenfalls weitgehend automatisch patientenbezogen einzustellen bzw. nachzustellen. Insbesondere soll die Einstellung bzw. Anpassung des durch die Beatmungseinrichtung vorgegebenen Drucks bei möglichst geringem Eingriff in den den Atemzyklus oder Beatmungszyklus (beide Begriffe werden im Folgenden synonym gebraucht) erfolgen können und möglichst keine oder jedenfalls nur geringfügige Eingriffe durch Ärzte oder Pflegepersonal erforderlich machen.

Zur Lösung dieser Aufgabe wird erfindungsgemäß ein System gemäß Anspruch 1 vorgeschlagen. Der durch die Beatmungseinrichtung vorgegebene Druck soll anhand des jeweils ermittelten transpulmonalen Drucks ermittelt werden, und zwar insbesondere derart, dass kein P/V-Manöver erforderlich ist, um den vorgegebenen Druck einzustellen.

Beispielsweise soll der PEEP anhand des am Ende der Expirationsphase ermittelten transpulmonalen Drucks ermittelt werden, und zwar insbesondere derart, dass kein P/V-Manöver erforderlich ist, um den PEEP einzustellen. Diese Vorgehensweise lässt auf elegante Weise eine Einstellung des PEEP zu, wobei der transpulmonale Druck am Ende der Expirationsphase immer positiv bleibt, jedenfalls nie deutlich unter Null sinkt.

In einem weiteren Beispiel kann, zusätzlich oder alternativ zum PEEP, der maximale Atemwegsdruck anhand des am Ende der Inspirationsphase ermittelten transpulmonalen Drucks ermittelt werden. Auch dies kann derart gesehen, dass kein P/V-Manöver erforderlich ist, um den maximalen Atemwegsdruck einzustellen. Beispielsweise kann eine Druckermittlungsanordnung zum Ermitteln eines transpulmonalen Drucks am Ende einer Inspirationsphase, sowie eine Einrichtung zur automatisierten Einstellung eines durch das Beatmungssystem vorgegebenen maximalen Atemwegsdrucksdrucks auf Grundlage des ermittelten transpulmonalen Drucks am Ende der Inspirationsphase vorgesehen sein. Die Druckermittlungsanordnung kann dieselbe sein wie die oben angesprochene Druckermittlungsanordnung zum Ermitteln eines transpulmonalen Drucks am Ende einer Expirationsphase. Bei der automatisierten Einstellung des durch das Beatmungssystem vorgegebenen maximalen Atemwegsdrucks kann zudem auch der transpulmonale Druck am Ende einer Expirationsphase sowie der eingestellte PEEP berücksichtigt werden. Beide Werte liegen auf den jeweiligen Patienten und die jeweilige Situation bezogen vor, wenn sie im Zuge einer patienten- und situationsbezogenen Bestimmung des PEEP auf Grundlage des transpulmonalen Drucks ermittelt werden. Die automatische Ermittlung und Einstellung des maximalen Atemwegsdrucks erlaubt es, Schäden am Lungengewebe durch zu hohen Atemwegsdruck während der Inspirationsphase auszuschließen. Auch hierbei kann berücksichtigt werden, dass für unterschiedliche Patienten unterschiedliche Obergrenzen für den Atemwegsdruck gelten können.

Zur Bestimmung des transpulmonalen Drucks kann die Druckermittlungsanordnung einerseits eine zur Bestimmung eines alveolären Drucks ausgelegte Sensorik und andererseits eine eine zur Bestimmung eines oesophagealen Drucks ausgelegte Sensorik aufweisen. Der jeweilige transpulmonale Druck lässt sich dann unter bestimmten Umständen anhand einer Differenz zwischen dem jeweiligen alveolären Druck und dem jeweiligem oesophagealen Druck ermitteln. Jeweilig soll bedeuten, dass die Bestimmung der alveolären bzw. oesophagealen Drücke am Ende einer Expirationsphase und/oder am Ende einer Inspirationsphase erfolgen soll. Es ist in verschiedenen Studien untersucht worden, inwieweit ein mittels einer in den Oesophagus eingeführten Sonde erfasster oesophagealer Druck hinreichend gut dem schwierig zu erfassenden Druck im Pleuraspalt entspricht, um näherungsweise zur Bestimmung des transpulmonalen Drucks herangezogen zu werden, siehe beispielsweise Talmor D. et al., New England Journal of Medicine, 2008, 359: S. 2095-2104. Die gemäß Weiterbildung der vorliegenden Erfindung vorgeschlagene Integration einer solchen Oesopha-gealdruckmessung in ein automatisiert arbeitendes System zur maschinellen Beatmung führt zu der Möglichkeit, Patienten mit einem individuell auf diese und deren Krankheitsverlauf abgestimmten PEEP und/oder maximalen Atemwegsdruck zu beatmen. Der PEEP und/oder der maximale Atemwegsdruck kann deswegen immer vergleichsweise hoch eingestellt sein mit den damit einhergehenden Vorteilen hinsichtlich Oxygenierung und effektiver Nutzung des gesamten Lungengewebes. Er ist aber niemals so hoch, dass hinsichtlich Überdehnung des Lungengewebes oder im Herz-Kreislaufsystem Beeinträchtigungen entstehen. Es hat sich erwiesen, dass die vorgeschlagene Oesophagealdruckmessung unter klinischen Bedingungen hinreichend gut handhabbar ist. Vor allem lässt sich aber auch eine ausreichend genaue und reproduzierbare Messung des Oesophagealdrucks in vielen Fällen ermöglichen, gerade wenn bei einer künstlichen Beatmung Patienten liegend in einer im Wesentlichen definierten Stellung verharren. Außerdem hat sich herausgestellt, dass der unter solchen Bedingungen gemessene Oesophagealdruck den tatsächlichen Wert des Drucks im Pleuraspalt hinreichend gut wiedergibt, um eine automatische Einstellung des PEEP und/oder des maximalen Atemwegsdrucks auf einem weitgehend optimalen Niveau zu erzielen.

Die Sensorik zur Bestimmung des oesophagealen Drucks kann beispielsweise einen zur Erfassung des oesophagealen Drucks in den Oesophagus einführbaren Katheter mit Ballonsonde umfassen. Solche Katheter sind beispielsweise bekannt aus Benditt J., Resp. Care, 2005, 50: S. 68-77.

Die Bestimmung des alveolären Drucks kann nach Maßgabe einer Bestimmung des Atemwegswiderstands sowie von Messungen des Gäsflusses im Atemweg am Ende der Expirationsphase und/oder am Ende der Inspirationsphase erfolgen. Aus diesen Daten, die mit Hilfe einer geeigneten Sensorik gut erfasst werden können, kann der jeweilige alveoläre Druck in recht guter Näherung ermittelt werden, wenn man zudem den Druck am Eingang des Atemwegs kennt. Messungen des Gasflusses sind bei Beatmungseinrichtungen häufig ohnehin vorhanden und können quasi in Echtzeit durchgeführt werden. Die Ermittlung des Atemwegswiderstands kann beispielsweise mittels statistischer Regressionsverfahren erfolgen, wie in Iotti I.A. et al., Intensive Care Med., 1995, 21: 406-413 beschrieben. Zur Bestimmung des Drucks am Eingang des Atemwegs kann ein am Tubusanfang angeordneter Atemwegsensor vorgesehen sein. Alternative könnte ein solcher Drucksensor auch einem Atemwegeingangsventil der Beatmungseinrichtung zugeordnet sein, so dass der eigentliche Tubus, der in der Regel nur einmal verwendbar ist, nicht über einen eigenen Drucksensor verfügen müsste. Der alveoläre Druck am Ende der Expirationsphase bzw. am Ende der Inspirationsphase ergibt sich dann näherungsweise aus einer Differenz zwischen dem jeweiligen Druck am Atemwegeingang und dem Produkt aus Atemwegswiderstand und Gasfluss am Ende der Expirationsphase/Gasfluss am Ende der Inspirationsphase.

Die voranstehend beschriebene Art der Bestimmung des alveolären Drucks lässt sich quasi in Echtzeit und ohne Unterbrechung des Atemzyklus oder Beatmungszyklus realisieren. Sie hat jedoch den Nachteil einer nur eingeschränkten Genauigkeit. Eine genauere Bestimmung des alveolären Drucks erlaubt eine Sensorik, die eine Anordnung zum Herstellen einer kurzzeitigen Okklusion des Atemwegs umfasst und derart ausgebildet ist, dass sie den alveolären Druck am Ende einer Expirationsphase und/oder den alveolären Druck am Ende einer Inspirationsphase anhand des während einer am Ende der Expirationsphase und/oder am Ende der Inpirationsphase stattfindenden Okklusion erfassten Drucks am Atemwegeingang bestimmt. Diese Art der Bestimmung des alveolären Drucks ist relativ genau. Sie hat jedoch den Nachteil, dass der Atemzyklus oder Beatmungszyklus während der Okklusion kurzzeitig unterbrochen werden muss. Die Okklusion sollte dabei so lange gewählt werden, dass ein Druckausgleich zwischen dem Druck in den Alveolen und dem Druck am Atemwegeingang erfolgen kann, also ein Moment erreicht wird, in dem der Gasfluss zum Stillstand kommt. Typischerweise dauert eine solche Okklusion zwischen 2 und 4 s. Ein derartiges Okklusionsmanöver kann in regelmäßigen Abständen, beispielsweise alle zwei Minuten oder alle ca. 20 Atemzüge, vorgesehen sein.

Eine kurzzeitige Okklusion der beschriebenen Art kann in einfacher Weise herbeigeführt werden, wenn sowohl ein Atemwegeingangsventil als auch ein Atemwegausgangsventil der Beatmungseinrichtung über einen vorbestimmten Okklusionszeitraum hinweg verschlossen werden. Beide Ventile sind ohnehin vorhanden und brauchen lediglich in entsprechend synchroner Weise angesteuert zu werden.

Die beiden voranstehend beschriebenen Arten der Bestimmung des alveolären Drucks lassen sich besonders gut miteinander kombinieren. Beispielsweise kann man so vorgehen, dass am Ende einer jeden Inspirationsphase bzw. am Ende einer jeden Expirationsphase (oder auch am Ende jeder zweiten, dritten, vierten, usw. Expirationsphase/Inspirationsphase) der alveoläre Druck anhand des jeweils herrschenden Gasflusses, des Atemwegswiderstands und des jeweils herrschenden Drucks am Atemwegeingang ermittelt wird. Sollte anhand des so ermittelten alveolären Drucks, bzw. des sich daraus ergebenden PEEP oder maximalen Drucks während der Inspirationsphase sich die Notwendigkeit der Einstellung eines neuen PEEP und/oder die Notwendigkeit der Einstellung eines neuen maximalen Atemwegsdrucks ergeben, so kann im nachfolgenden Atemzyklus oder Beatmungszyklus eine Bestimmung des alveolären Drucks anhand einer am Ende der Expirationsphase bzw. am Ende der Inspirationsphase erfolgenden Okklusion erfolgen und der neue PEEP und/oder der neue maximale Atemwegsdruck dann unter Berücksichtigung der genaueren Ergebnisse dieser Bestimmung erfolgen. Damit kann Atemzug für Atemzug überwacht werden, ob der eingestellte PEEP und/oder der eingestellte maximale Atemwegsdruck aufgrund von Veränderungen von physiologischen Parametern, insbesondere des transpulmonalen Drucks, eines jeweiligen Patienten verändert werden sollte. Es sind nur minimale Eingriffe in den Atemzyklus oder Beatmungszyklus des Patienten erforderlich, weil erst dann, wenn tatsächlich eine Veränderung des PEEP und/oder des maximalen Atemwegsdrucks angezeigt ist, eine kurzzeitige Okklusion am Ende der Expirationsphase bzw. am Ende der Inspirationsphase erforderlich ist. Solange der eingestellte PEEP und/öder der eingestellte maximale Atemwegsdruck überwacht wird, ohne dass Veränderungen angezeigt sind, brauchen keinerlei Eingriffe in den Atemzyklus oder Beatmungszyklus erfolgen.

Es ist bei Anwendung des vorgeschlagenen Systems ohne Weiteres denkbar, dass in einem jeweiligen Atemzyklus oder Beatmungszyklus der PEEP und/oder der maximale Atemwegsdruck für den nachfolgenden Atemzyklus ermittelt wird, der PEEP und/oder der maximale Atemwegsdruck also atemzugweise überwacht und ggf. neu eingestellt wird. Die Neueinstellung des PEEP und/oder des maximalen Atemwegsdrucks kann nach oben oder nach unten erfolgen.

Die Neueinstellung des PEEP und/oder des maximale Atemwegsdrucks erfolgt dabei nicht anhand von vorgegebenen Richtwerten, sondern auf Grundlage von physiologischen Parametern, die für den Patienten individuell und aktuell ermittelt werden, insbesondere auf Grundlage des für den Patienten individuell und aktuell ermittelten transpulmonalen Drucks.

Um allzu häufige Änderungen des PEEP und/oder des maximalen Atemwegsdrucks zu vermeiden, kann man den PEEP und/oder den maximalen Atemwegsdruck für den nachfolgenden Atemzyklus nicht nur anhand des aktuellen Atemzyklus ermitteln, sondern die in mehreren vorangehenden Atemzyklen ermittelten Werte des PEEP und/oder des maximalen Atemwegsdrucks zugrunde legen. Dadurch lassen sich Messungenauigkeiten bzw. statische Schwankungen ausgleichen, so dass der PEEP und/oder der maximale Atemwegsdruck erst dann neu eingestellt wird, wenn die physiologische Situation des Patienten einen tatsächlichen Trend zeigt. Ein ähnlicher Effekt lässt sich erzielen durch einen zusätzlich oder alternativ durchgeführten Vergleich des für den nachfolgenden Atemzyklus ermittelten PEEP und/oder maximalen Atemwegsdrucks mit dem für den aktuellen Atemzyklus eingestellten PEEP und/oder maximalen Atemwegsdruck, mit der Maßgabe, dass der PEEP und/oder der maximale Atemwegsdruck nur dann verändert wird, wenn die Differenz zwischen beiden Werten einen vorbestimmten Schwellenwert übersteigt.

Man kann beispielsweise so vorgehen, dass am Ende eines jeweiligen Atemzyklus anhand des Gasflusses im Atemweg am Ende der Expirationsphase und/oder am Ende der Inspirationsphase sowie anhand des Atemwegswiderstands ein vorläufiger Wert für den PEEP und/oder den maximalen Atemwegsdruck im nachfolgenden Atemzyklus bestimmt wird. Nur dann, wenn der vorläufige Wert für den PEEP und/oder den maximale Atemwegsdruck im nachfolgenden Atemzyklus sich vom dem eingestellten Wert für den PEEP und/oder den maximale Atemwegsdruck um einen vorbestimmten Schwellenwert oder mehr unterscheidet, wird im nachfolgenden Atemzyklus eine kurzzeitige Okklusion durchgeführt und ein neuer Wert für den PEEP und/oder den maximale Atemwegsdruck bestimmt.

Das vorgeschlagene System kann durchaus von vorgegebenen Richtlinien Gebrauch machen, wie sie beispielsweise in den oben beschriebenen ARDSnet-Richtlinien festgelegt sind. Eine Möglichkeit besteht darin, von solchen Richtlinien auszugehen, wobei der Wert des PEEP in einem nachfolgenden Atemzyklus nach Maßgabe einer Abweichung des in einem oder mehreren vorangehenden Atemzyklen ermittelten transpulmonalen Drucks am Ende einer Expirationsphase von einem normierten transpulmonalen Druck am Ende einer Expirationsphase bestimmt wird.

Beispielsweise kann die Ermittlung des Werts für den PEEP im nachfolgenden Atemzyklus gemäß folgender Formel erfolgen:

$$PEEP\_n+1 = PEEP\_n + f * (Ptp\_ee\_norm - Ptp\_ee\_n)$$

mit:

PEEP_n+1:      PEEP im nachfolgenden Atemzyklus
PEEP_n:      PEEP im aktuellen Atemzyklus oder PEEP in m vorangehenden Atemzyklen
Ptp_ee_norm:      normierter transpulmonaler Druck am Ende der Expirationsphase,
Ptp_ee_n:      im aktuellen Atemzyklus oder in m vorangehenden Atemzyklen bestimmter transpulmonaler Druck am Ende der Expirations phase, mit $m \geq 1$,
$0 \leq f \leq 1$,      insbesondere $0.01 \leq f \leq 0.8$, insbesondere $0.05 \leq f \leq 0.5$, insbesondere $f = 0.2$.

Der normierte transpulmonale Druck am Ende der Expirationsphase lässt sich beispielsweise anhand einer vorbestimmten Beziehung zwischen dem normierten transpulmonalen Druck am Ende der Expirationsphase und der Sauerstofffraktion der Atemgas ermitteln. Derartige Beziehungen sind beispielsweise in den bereits erwähnten ARDSnet-Richtlinien in Form von entsprechenden Tabellen niedergelegt.

Ein Startwert für den PEEP lässt sich etwa anhand einer vorbestimmten Beziehung zwischen dem PEEP. und der Sauerstofffraktion der Atemgas FiO2 ermitteln. In der Vergangenheit hat sich gezeigt, dass eine derartige vorbestimmte Beziehung häufig davon abhängig ist, ob die Sauerstofffraktion der Atemgas FiO2 gegenüber der seither eingestellten Sauerstofffraktion erhöht oder verringert wird, so dass es ratsam ist, diesen Trend der Veränderung der Sauerstofffraktion der Atemgas bei der Auswahl der geeigneten Beziehung zu berücksichtigen.

Um den maximalen Atemwegsdruck in dem nachfolgenden Atemzyklus zu bestimmen, ist günstig, eine Differenz zwischen dem in einem oder mehreren vorangehenden Atemzyklen ermittelten Atemwegsdruck am Ende einer Inspirationsphase und dem in einem oder mehreren vorangehenden Zyklen angewendeten PEEP zu bestimmen.

Der maximale Atemwegsdruck in dem nachfolgenden Atemzyklus lässt sich dann nach folgender Formel bestimmen:

$$Paw\_max\_n+1 = [(Paw\_ei\_n - PEEP\_n)/(Ptp\_ei\_n - Ptp\_ee\_n)] * Ptp\_ei\_max$$

mit:

Paw_max_n+1:    maximaler Atemwegsdruck im nachfolgenden Atemzyklus;

Ptp_ei_max:    vorbestimmter maximaler transpulmonaler Druck am Ende der Inspirationsphase;

Ptp_ei_n:    im aktuellen Atemzyklus oder in m vorangehenden Atemzyklen bestimmter transpulmonaler Druck am Ende der Inspirations phase;

Ptp_ee_n:    im aktuellen Atemzyklus oder in m vorangehenden Atemzyklen bestimmter transpulmonaler Druck am Ende der Expirations phase;

Paw_ei_n:    im aktuellen Atemzyklus oder in m vorangehenden Atemzyklen bestimmter Atemwegsdruck am Ende der Inspirationsphase;

PEEP_n    im aktuellen Atemzyklus oder in m vorangehenden Atemzyklen bestimmter PEEP

mit $m \geq 1$.

Der maximale transpulmonale Druck am Ende der Inspirationsphase ist dabei beispielsweise anhand von vorgegebenen Standardwerten festzulegen. Solche Standardwerte können zwischen 15 und 20 cm $H_2O$ liegen.

Ein geeigneter Startwert für den maximalen Atemwegsdruck am Ende der Inspirationsphase lässt sich ebenfalls anhand von vorgegebenen Standardwerten ermitteln. Als ein gut geeigneter Startwert hat sich ein maximaler Atemwegsdruck am Ende der Inspirationsphase von 30 cm $H_2O$ erwiesen.

Die Erfindung betrifft darüber hinaus eine Einrichtung zur maschinellen Beatmung von Patienten, umfassend ein System zur automatischen Einstellung eines von der Beatmungseinrichtung vorgegebenen Drucks, insbesondere eines positiven endexpiratorischen Drucks und/oder eines maximalen Atemwegsdrucks, wie vorangehend beschrieben. Die Beatmungseinrichtung arbeitet dabei insbesondere nach dem Überdruckprinzip, wobei die Beatmung druckgesteuert oder volumengesteuert erfolgen kann und auch Mischformen möglich sind. Die Beatmungseinrichtung kann im Prinzip alle bekannten Beatmungsformen aufweisen, neben Beatmungsformen mit totaler Übernahme der Beatmung durch die Maschine sind auch Beatmungsformen denkbar, bei denen die Beatmungseinrichtung die Eigenatmung des Patienten durch intermittierende maschinelle Beatmungszüge unterstützt und/oder einzelne Spontanatmungszüge des Patienten unterstützt.

Die Erfindung wird nachfolgend unter Bezugnahme auf die Zeichnungen anhand von Ausführungsbeispielen im Detail erläutert. Es zeigt:

Fig. 1: In stark schematisierter Darstellung die wesentlichen Elemente einer Beatmungseinrichtung samt intubierter Trachea und Thorax eines beatmeten Patienten;

Fig. 2: den zeitlichen Verlauf des Atemwegeingangsdrucks Paw (oben), oesophagealen Drucks Peso (Mitte) und der Differenz Paw - Peso aus beiden Drücken während mehrerer aufeinander folgender Atemzyklen bei maschineller Beatmung einschließlich Okklusionsmanövern;

Fig. 3: ein Flussdiagramm zur Bestimmung des PEEP während eines Atemzyklusses bei maschineller Beatmung;

Fig. 4a und 4b Diagramme, die eine Beziehung zwischen Sauerstoffanteil des Atemgases FiO2 und PEEP zeigen; und

Fig. 5: eine Tabelle, die eine Beziehung zwischen einem normierten transpulmonalen Druck am Ende einer Expirationsphase und dem Sauerstoffanteil des Atemgases FiO2 zeigt.

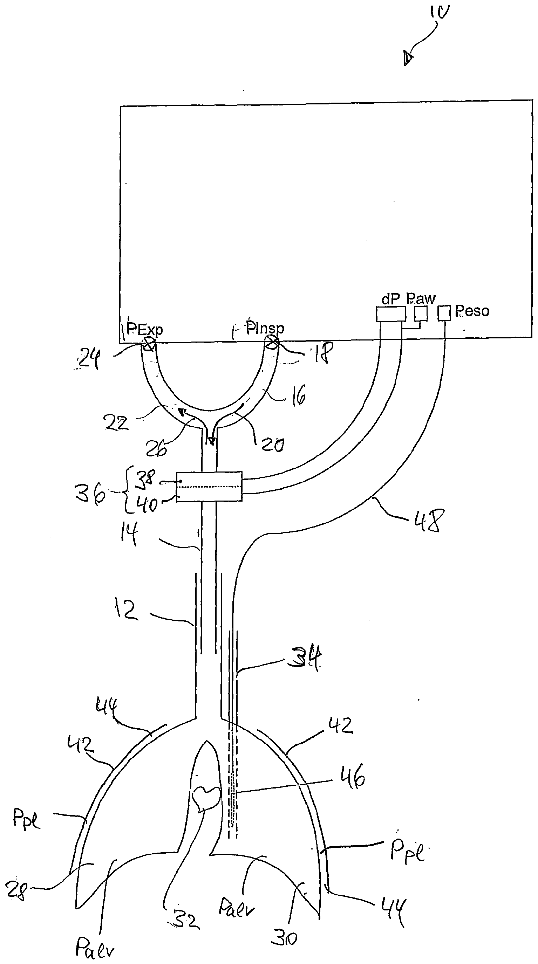

Fig. 1 zeigt in stark schematisierter Darstellung und in Form eines Blockdiagramms die wesentlichen Elemente einer Beatmungseinrichtung 10. Die Beatmungseinrichtung 10 ist in Fig. 1 in einem Zustand mit intubierter Luftröhre (Trachea) 12 eines beatmeten Patienten gezeigt. Neben der Trachea 12 sind in Fig. 1 noch die Lungenlappen 28, 30, das Herz 32, die Speiseröhre (Ösophagus) 34 und die Thoraxwand 42 des Patienten sehr schematisch angedeutet. Der Tubus 14 der Beatmungseinrichtung 10 ist, in der Regel über die nicht gezeichnete Mundöffnung des Patienten, ein Stück weit in die Trachea 12 eingeschoben, um den Atemweg mit Atemgas zu beaufschlagen. Ausgeatmete Luft wird ebenfalls über den Tubus 14 abgeleitet, der sich an seinem stromaufwärtigen Ende in ein erstes Ende 16 und ein zweites Ende 22 verzweigt. Das erste Ende 16 ist über ein Atemwegeingangsventil 18 mit einem Atemwegeingangsanschluss der Beatmungseinrichtung 10 zur Beaufschlagung eines Inspirationsdrucks

PInsp verbunden. In Öffnungsstellung des Atemwegeingangsventils 18 ist der Atemweg mit dem Inspirationsdruck PInsp beaufschlagt. Das zweite Ende 22 ist über ein Atemwegausgangsventil 24 mit einem Atemwegausgangsanschluss der Beatmungseinrichtung 10 zur Beaufschlagung eines Expirationsdrucks PExp verbunden. In Öffnungsstellung des Atemwegausgangsventils 24 ist der Atemweg mit dem Expirationsdruck PExp beaufschlagt.

Sowohl der Inspirationsdruck PInsp als auch der Expirationsdruck PExp werden von der Beatmungseinrichtung 10 nach vorgegebenen zeitlichen Mustern erzeugt, derart dass einzuatmende Atemgas während einer Inspirationsphase in Richtung der Lunge 28, 30 des Patienten strömt, wie durch den Pfeil 20 in Fig. 1 angedeutet, und während einer Expirationsphase das auszuatmende Atemgas von der Lunge 28, 30 des Patienten zurückströmt, wie durch den Pfeil 26 angedeutet. Normalerweise bleibt während der Inspirationsphase das Atemwegeingangsventil 18 geöffnet und der Atemwegeingang wird mit dem Inspirationsdruck PInsp - dieser ist in der Regel größer als der Expirationsdruck PExp - beaufschlagt. Während der Expirationsphase ist das Atemwegeingangsventil 18 geschlossen und das Atemwegausgangsventil 24 ist geöffnet. Dann ist der Atemwegeingang mit dem Expirationsdruck PExp beaufschlagt.

Im Zusammenhang mit der vorliegenden Erfindung sind jegliche Formen bekannter Beatmungsmuster einsetzbar, beispielsweise druckkontrollierte Beatmungsformen, volumenkontrollierte Beatmungsformen oder auch Beatmungsformen, bei denen druckkontrollierte und volumenkontrollierte Aspekte kombiniert sind. Neben rein maschinenkontrollierten Beatmungsformen, bei denen der zeitliche Verlauf des Inspirationsdrucks PInsp und ggf. auch des Expirationsdrucks PExp von der Beatmungseinrichtung 10 bestimmt werden, sind auch Beatmungsformen denkbar, in denen Spontanätmungsbemühungen des Patienten entweder die maschinelle Beatmung unterstützten können oder die maschinelle Beatmung zur Unterstützung von Spontanatmungsbemühungen des Patienten dient. In solchen Beatmungsformen werden der zeitliche Verlauf von Inspirationsdruck PInsp bzw. Expirationsdruck PExp sowie häufig ebenfalls die Stellung von Eingangsventil 18 bzw. Ausgangsventil 24 nicht allein von der Beatmungseinrichtung 10 vorgegeben, sondern von Spontanatmungsbemühungen des Patienten mit beeinflusst.

Das Atemgas kann Umgebungsluft enthalten, wird aber in der Regel einen vorbestimmten Anteil reinen Sauerstoff, im Folgenden als $FiO_2$ bezeichnet, enthalten, der über dem Sauerstoffanteil der Umgebungsluft liegt. Das Atemgas wird in der Regel außerdem befeuchtet sein.

Der Fluss des Atemgases am Atemwegeingang wird mit Hilfe eines Atemwegeingangsflusssensors 36 bestimmt. Der Atemwegeingangsflusssensor 36 beruht auf der Erfassung einer Druckdifferenz dP zwischen einem Eingangsvolumen 38 und einem mit dem Eingangsvolumen 38 in Verbindung stehenden Ausgangsvolumen 40, und liefert eine Bestimmung des Atemgasmassenflusses am Atemwegeingang. Aus dem Drucksignal im Ausgangsvolumen 40 lässt sich zugleich recht einfach der Wert des Atemwegeingangsdrucks Paw ableiten.

Der in den Alveolen der Lunge 28, 30 herrschende Druck ist in Fig. 1 mit Palv angedeutet. Dieser Druck hängt ab vom Atemwegeingangsdruck Paw sowie der Strömung des Atemgases V in die Lunge bzw. aus der Lunge heraus und dem Atemwegswiderstand R. Im Falle eines Druckausgleichs zwischen Atemwegeingang und Alveolen ist der alveoläre Druck Palv gleich dem Atemwegeingangsdruck. Ein derartiger Druckausgleich hat zur Konsequenz, dass der Atemgasfluss V zum Erliegen kommt. Beispielsweise kann ein kurzzeitiges Okklusionsmanöver des Atemwegs, d.h. Atemwegeingangsventil 18 und Atemwegausgangsventil 24 bleiben gleichzeitig geschlossen, zu einem Druckausgleich führen. Dabei muss das Okklusionsmanöver gerade solange dauern, dass der Gasfluss V im Atemweg zum Erliegen kommt. Das sind in der Regel zwischen 1 und 5 s. In diesem Zustand kann dann der alveoläre Druck Palv mittels einer Bestimmung des Atemwegeingangsdrucks Paw bestimmt werden.

Sowohl bei der physiologischen Atmung als auch bei maschineller Beatmung wird der Fluss des Atemgases bestimmt durch eine Druckdifferenz zwischen dem alveolären Druck Palv und dem Atemwegeingangsdruck Paw.

Im Falle der rein physiologischen Atmung wird zum Einatmen eine negative Druckdifferenz, d.h. ein Unterdruck, zwischen dem alveolären Druck Palv und dem Atemwegeingangsdruck Paw erzeugt durch Ausdehnung des Thorax (angedeutet bei 42 in Fig. 1) und damit verbundener Absenkung des Drucks Ppl in dem zwischen Thorax 42 und Lunge 28, 30 gebildeten Pleuraspalt 44. Die Ausatmung erfolgt passiv durch Entspannen des Thorax und elastischer Rückstellung des Lungengewebes. Aus diesem Grund ist bei physiologischer Atmung der Druck im Pleuraspalt Ppl immer kleiner als der alveoläre Druck Palv. Der als Differenz zwischen dem alveolären Druck Palv und dem Druck im Pleuraspalt Ppl definierte transpulmonale Druck Ptp ist damit generell positiv und wird Null im Falle eines vollständigen Druckausgleichs.

Bei maschineller Beatmung wird das Atemgas mit Überdruck in die Lunge gepumpt. Aus diesem Grund ist bei maschineller Beatmung während der Inspirationsphase der Atemwegeingangsdruck Paw = PInsp größer als der alveoläre Druck Palv und dieser wiederum größer als der Druck im Pleuraspalt Ppl. Aus diesen Druckverhältnissen folgt, dass der transpulmonale Druck Ppl bei maschineller Beatmung während der Inspiration positiv ist. Während der Expiration wird der Atemwegeingang mit einem Atemwegsdruck PExp beaufschlagt, der geringer ist also der alveoläre Druck Palv, so dass Atemgas aus den Alveolen herausströmt. Im Falle eines sehr kleinen Atemwegsdrucks PExp kann es vorkommen, dass am Ende der Expiration, wenn nur noch sehr wenig Gas in der Lunge vorhanden ist, der Druck im Pleuraspalt Ppl den alveolären Druck Palv in so hohem Maße übersteigt, dass ein Teil der Alveolen der Lunge kollabiert. Der transpulmonale Druck Ptp ist dann negativ.

Das Kollabieren der Alveolen lässt sich verhindern, wenn man auch in der Expirationsphase den Atemwegeingang mit einem zusätzlichen positiven Druck beaufschlagt. Es liegt dann am Atemwegeingang dauerhaft, d.h. während der Inspirationsphase und auch während der Expirationsphase ein positiver Atemwegsdruck an. Dieser positive Atemwegsdruck wird als positiver endexpiratorischer Druck oder PEEP bezeichnet.

Der transpulmonale Druck Ptp ist demzufolge eine geeignete Größe zur Einstellung des PEEP; Allerdings ist der transpulmonale Druck Ptp einer unmittelbaren Erfassung nicht zugänglich und lässt sich auch nicht aus den bei maschineller Beatmung regelmäßig erfassten Drücken, wie sie oben beschrieben sind, ermitteln.

In Fig. 1 ist in schematischer Weise eine zusätzliche Drucksonde 46 zur Messung des als oesophagealer Druck Peso bezeichneten Drucks in der Speiseröhre (Trachea) 34 angedeutet. Die Sonde 46 ist in Form einer sogenannten Ballonsonde an einem in die Speiseröhre 34 eingeführten Katheter 48 angebracht. Die Ballonsonde 46 liegt innen an der Wand der Speiseröhre 34 an und liefert den in auf die Speiseröhre am Ort der Ballonsonde 46 einwirkenden Druck. Dieser Druck entspricht bei geeigneter Lagerung des Patienten in guter Näherung dem Druck Ppl im Pleuraspalt. Die beschriebene Ballonsonde 46 zur Erfassung des oesophagealen Drucks Peso und der Umgang mit dieser Sonde ist in Benditt J., Resp. Care, 2005, 50: S. 68-77 beschrieben.

Will man den transpulmonalen Druck Ptp bestimmen, so benötigt man neben dem Druck im Pleuraspalt Ppl noch Information über den alveolären Druck Palv. Eine recht elegante Möglichkeit zur Bestimmung des alveolären Drucks zu einem bestimmten Zeitpunkt t bietet die Erfassung des Atemgasflusses V(t), die mit Hilfe des Flusssensors 36 vorgenommen werden kann. Dann kann man auf den alveolären Druck zum Zeitpunkt t schließen nach der Beziehung: Palv(t) = Paw (t) - R * V(t), wobei R den Atemwegswiderstand bezeichnet. Der Atemwegswiderstand ist für ein und denselben Patienten eine sich im Wesentlichen nicht oder nur vergleichsweise langsam ändernde Größe und kann nach im Stand der Technik bekannten Methoden bestimmt werden. Beispielsweise sei verwiesen auf Iotti I.A. et al., Intensive Care Med., 1995, 21: 406-413. Da es zur Bestimmung eines geeigneten PEEP vor allem auf den transpulmonalen Druck am Ende der Expirationsphase Ptp_ee ankommt, wird man im Zusammenhang mit einer automatischen Einstellung des PEEP eine Bestimmung des alveolären Drucks Palv vorzugsweise am Ende der Expirationsphase vornehmen, gemäß der Formel:

$$Ptp\_ee = Palv\_ee - Peso\_ee = Paw\_ee - R * V\_ee - Peso\_ee.$$

Der PEEP sollte dann so eingestellt werden, dass Ptp_ee immer positiv bleibt, jedenfalls nie deutlich unter Null sinkt.

Leider lässt die beschriebene Methode der Bestimmung des alveolären Drucks Palv, die recht einfach in einer automatisiert arbeitenden Beatmungseinrichtung 10 realisierbar ist, nur eine vergleichsweise grobe Abschätzung des geeigneten PEEP zu. Das liegt vor allem an dem nur recht ungenau abschätzbaren Atemwegswiderstand R, der zudem in der Regel im zeitlichen Verlauf einer Therapie durchaus einem gewissen Trend unterworfen sein wird.

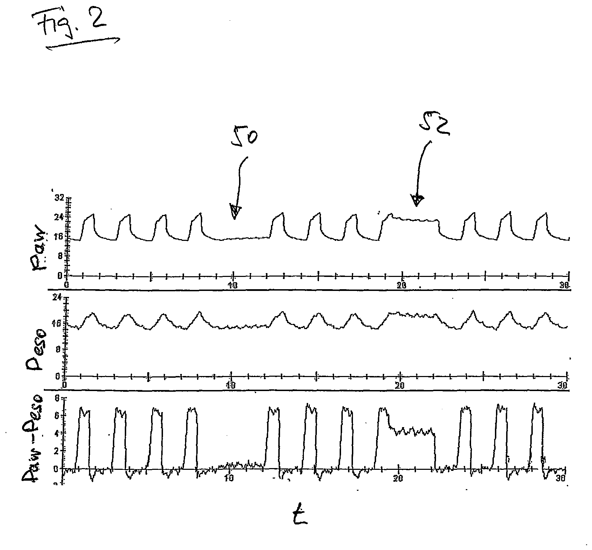

Eine alternative Methode zur Bestimmung des alveolären Drucks Palv beruht auf einem kurzzeitigen Okklusionsmanöver, bei dem sowohl das Atemwegeingangsventil 18 als auch das Atemwegausgangsventil 24 gleichzeitig geschlossen bleiben. In diesem Okklusionszustand tritt ein Ausgleich der im Atemweg herrschenden Drücke ein. Führt man ein solches Okklusionsmanöver am Ende einer Expirationsphase durch, so ist der sich nach einer genügend längen Okklusion im Atemweg einstellende Druck in guter Näherung gleich dem alveolären Druck Palv am Ende der Expirationsphase. Dieser Druck kann dann recht einfach mit Hilfe der am Atemwegeingang angeordneten Drucksonde zur Messung des Atemwegsdrucks Paw erfasst werden. In Fig. 2 ist diese Situation an der mit 50 bezeichneten Stelle dargestellt.

Fig. 2 zeigt übereinander jeweils den zeitlichen Verlauf des Atemwegdrucks Paw (oben), des mit der Ballonsonde 46 gemessenen oesophagealen Drucks Peso (Mitte) und der Differenz Paw - Peso aus beiden Drücken während mehrerer aufeinander folgender Atemzyklen, zwischen denen auch Okklusionsmanöver durchgeführt wurden. Man kann deutlich die einzelnen Atemzyklen erkennen, mit je einer Inspirationsphase (hoher ansteigender Atemwegdruck Paw) und einer Expirationsphase (abfallender Atemwegdruck Paw). Der oesophageale Druck Peso folgt dem Atemwegdruck Paw, allerdings in abgeschwächter Form. Der in der unteren Kurve dargestellte Differenzdruck Paw - Peso würde - bei genügend langsamer Strömung des Atemgases, so dass immer ein Druckausgleich stattfindet - recht gut dem transpulmonalen Druck Ptp entsprechen. Diese Voraussetzung ist in der Praxis jedoch wegen der sich stark ändernden Atemgasströmung nicht der Fall, abgesehen an den mit 50 und 52 bezeichneten Stellen, bei denen eine kurzzeitige Ocklusion am Ende einer Expirationsphase durchgeführt wurde (50, zwischen etwa 8 s und 12 s) bzw. eine kurzzeitige Okklusion am Ende einer Inspirationsphase durchgeführt wurde (52, zwischen etwa 18,5 s und 24,5 s). Die Okklusion dauerte in beiden Fällen etwa 4 s. Das entspricht in dem gewählten Beispiel in etwa der Dauer eines Atemzyklus. Generell sollte die Okklusion so lange dauern, dass ein Druckausgleich im Atemweg stattfindet und der Gasfluss im Atemweg damit zum Erliegen kommt.

Am Ende der mit 50 bezeichneten Stelle des zeitlichen Profils (etwa zwischen 11 s und 12 s, beispielsweise in den letzten ca. 200 ms der Okklusion) entspricht der in der dritten Zeile in Fig. 2 dargestellte Druck Paw - Peso in recht guter Näherung dem transpulmonalen Druck am Ende der Expirationsphase Ptp_ee. Zur Bestimmung von Ptp_ee könnte man beispielsweise den Mittelwert von Paw - Peso über den genannten Zeitraum hinweg bilden. Am Ende der mit 52 bezeichneten Stelle des zeitlichen Profils (etwa zwischen 21,5 s und 22,5 s, beispielsweise in den letzten ca. 200 ms der Okklusion) entspricht der in der dritten Zeile dargestellte Druck Paw - Peso in recht guter Näherung dem transpulmonalen Druck am Ende der Inspirationsphase Ptp_ei. Zur Bestimmung von Ptp_ei könnte man beispielsweise den Mittelwert von Paw - Peso über den genannten Zeitraum hinweg bilden.

Die Bestimmung des transpulmonalen Drucks Ptp mit Hilfe des beschriebenen Okklusionsmanövers ist genauer als die oben beschriebene Methode mit Hilfe des Atemwegswiderstands R. Allerdings erfordert sie das Durchführen eines Okklusionsmanövers am Ende einer Expirationsphase bzw. am Ende einer Inspirationsphase. Deshalb stört diese Methode naturgemäß den Atemzyklus, und zwar umso erheblicher als die Dauer der Okklusion im Vergleich zur Dauer des Atemzyklus ist. Aus diesem Grund ist es ratsam, so vorzugehen, dass man recht häufig, beispielsweise nach jedem Atemzug oder alle n Atemzüge (n>1), mit Hilfe der Atemwegswiderstandsmethode überprüft, ob ein eingestellter Wert des PEEP und/oder ein eingestellter Wert des maximalen Atemwegsdrucks noch im Rahmen der Vorgaben liegt oder ob ein sich ergebender Wert des transpulmonalen Drucks Ptp_ee noch innerhalb bestimmter Vorgaben für einen normierten transpulmonalen Druck Ptp_ee_ideal liegt. Sollte sich bei dieser Prüfung herausstellen, dass dies nicht der Fall ist und deswegen ein neuer (höherer oder niedrigerer) Wert für den PEEP und/oder den maximalen Atemwegsdruck eingestellt werden sollte, so wird im folgenden Atemzyklus ein Okklusionsmanöver am Ende der Expirationsphase durchgeführt und der neue Wert für den PEEP anhand dieser Okklusion wie oben beschrieben ermittelt. Alternativ könnte man auch das Okklusionsmanöver wie beschrieben alle n Atemzyklen wiederholen, mit n > 1, beispielsweise n = 10, 50, oder 100.

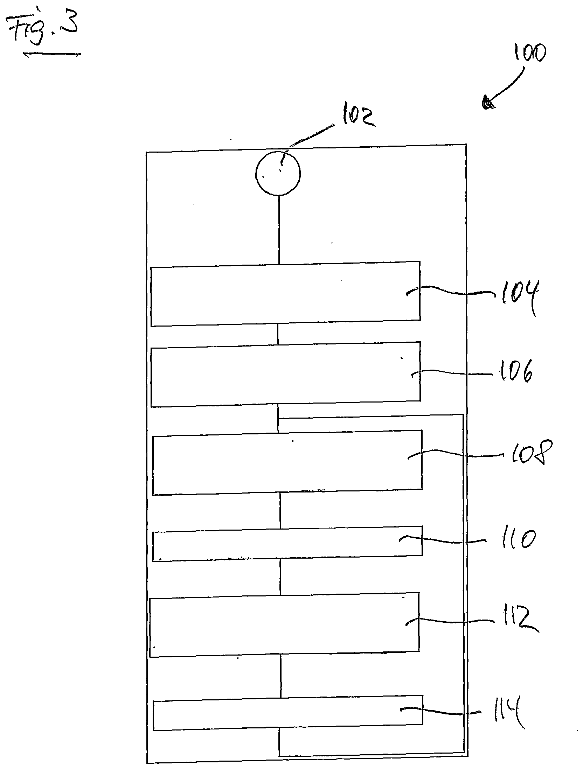

Fig. 3 zeigt ein Flussdiagramm zur Bestimmung des PEEP und des maximalen Atemwegsdrucks während der maschinellen Beatmung. Dieses Flussdiagramm wird während der Beatmung kontinuierlich abgewickelt.

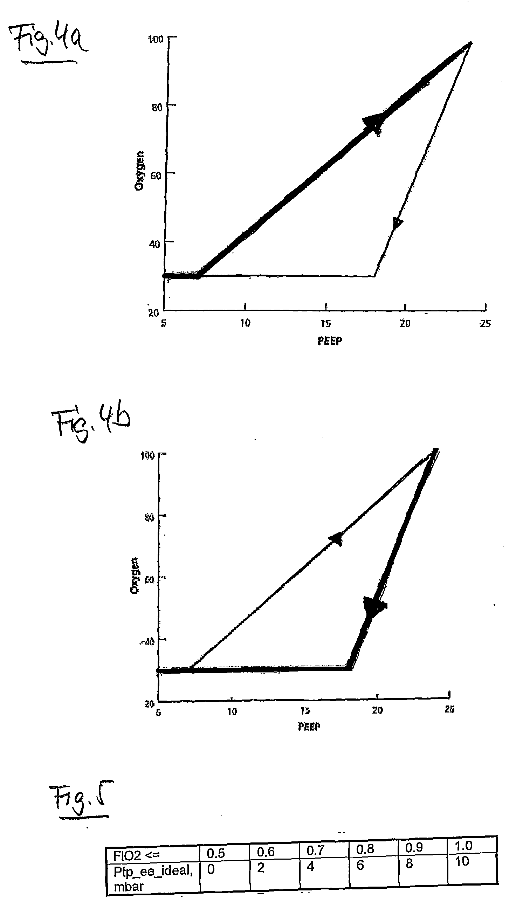

Die Prozedur 100 in Fig. 3 startet in Schritt 102. Danach folgt in Schritt 104 die Bestimmung eines vorgegebenen PEEP. Diese Bestimmung erfolgt anhand des für den Patienten nach Diagnose bzw. Therapie vorgewählten Anteils von Sauerstoff in der Atemgas FiO2. Solche Beziehungen zwischen Sauerstoffanteil des Atemgases FiO2 und PEEP sind in früheren Studien ermittelt und veröffentlicht worden, beispielsweise in den bereits oben angesprochenen ARDSnet-Richtlinien, The Acute Respiratory Distress Syndrome Network, The New England Journal of Medicine, 2000, 342: S. 1301-1308 oder Grasso et al., American Journal of Respiratory Critical Care, 2007, 176: S. 761-767. Es wird häufig eine unterschiedliche Beziehung zwischen eingestelltem FiO2 und einzustellendem PEEP verwendet, je nachdem, ob der Fi02-Wert seit dem letzten eingestellten FiO2-Wert erhöht worden ist ("zunehmende Therapie") oder verringert worden ist ("abnehmende Therapie"). Bei zunehmender Therapie wird in der Regel für einen gegebenen FiO2-Wert ein kleinerer PEEP eingestellt als bei abnehmender Therapie. Dies ist beispielsweise in den Fig. 4a und 4b gezeigt, wo in Fig. 4a die Beziehung zwischen dem Anteil Sauerstoff im Atemgas FiO2 und PEEP für zunehmende Therapie gezeigt ist und in Fig. 4b diese Beziehung für abnehmende Therapie gezeigt ist. In beiden Fig. 4a und 4b ist die jeweils dick gezeichnete Linie maßgeblich, wie auch durch den Pfeil angedeutet. Die dünne Linie dient nur zum Vergleich mit der jeweils anderen Linie.

In Schritt 106 erfolgt dann eine (provisorische) Festlegung eines maximalen Atemwegsdrucks PInsp_max während der Inspirationsphase. Der maximale Atemwegsdruck während der Inspirationsphase Plnsp_max ist in der Regel auch der größte überhaupt anliegende Atemwegsdruck Paw_max. Dieser Druck wird so vorgegeben, dass bestimmte kritische Werte hinsichtlich Überdehnüngder Lunge nicht überschritten werden. Ein häufig gewählter Wert ist Paw_max = 30 cm $H_2O$.

In Schritt 108 werden der Atemwegsdruck am Ende der Expirationsphase Paw_ee und der oesophageale Druck am Ende der Expirationsphase Peso_ee erfasst. Dies kann beispielsweise nach der oben beschriebenen Methode unter Zuhilfenahme der Atemwegswiderstands R geschehen oder während einer endexpiratorischen Okklusion wie oben in Bezug zu Fig. 2 beschrieben. Anhand der gemessenen Werte von Paw_ee_n und Peso_ee_n wird dann ein neuer Wert für den PEEP im nachfolgenden Atemzyklus bestimmt. Zur Bestimmung des neuen PEEP kann wie folgt vorgegangen werden: Aus früheren Studien ist eine Beziehung zwischen dem für einen Patienten vorgewählten Anteil an Sauerstoff im Atemgas FiO2 und einem normierten (oder idealen) transpulmonalen Druck am Ende einer Expirationsphase Ptp_ee_ideal bekannt. Diese Beziehung kann beispielsweise aussehen, wie in Fig. 5 dargestellt. Aus den am Ende einer Expirationsphase bestimmten Werten von Paw_ee_n und Peso_ee_n wird dann ein tatsächlicher transpulmonaler Druck am Ende der Expirationsphase Ptp_ee_n bestimmt. Der im folgenden Atemzyklus einzustellende neue PEEP wird dann anhand einer Abweichung des tatsächlichen transpulmonalen Drucks Ptp_ee von dem dem jeweiligen FiO2 zugeordneten normierten transpulmonalen Druck am Ende einer Expirationsphase Ptp_ee_ideal bestimmt, und zwar gemäß der Formel:

$$\text{PEEP\_n+1} = \text{PEEP\_n} + f * (\text{Ptp\_ee\_ideal} - \text{Ptp\_ee\_n}).$$

Hierbei ist f ein geeigneter Dämpfungsfaktor, der in der Regel zwischen 0 und 1 gewählt wird, insbesondere zwischen 0.1 und 0.8 und bevorzugt zwischen 0.05 und 0,5. In dem beschriebenen Beispiel gilt f = 0.2.

Um Schwankungen zu unterdrücken, kann statt den im aktuellen Atemzyklus gemessenen Werten von Ptp_ee_n bzw. PEEP_n auch ein Mittelwert aus in m vorangehenden Atemzyklen bestimmten Werten zugrunde gelegt werden, mit m >1.

In Schritt 110 werden eine vorbestimmte Anzahl z von Atemzyklen abgewartet, wobei gilt z ≥ 1. Alternativ kann auch eine vorbestimmte Zeit abgewartet werden und die Prozedur mit dem nach Ablauf dieser Zeit beginnenden Atemzyklus weitergeführt werden.

In Schritt 112 werden der Atemwegsdruck am Ende einer Inspirationsphase Paw_ei und der oesophageale Druck am Ende einer Inspirationsphase Peso_ei erfasst. Peso_ei wird wie oben beschrieben mit Hilfe der Ballonsonde 34 bestimmt. Paw_ei kann nach der beschriebenen Methode unter Zuhilfenahme des Atemwegsflüsse am Ende der Inspirationsphase V_ei und des Atemwegswiderstands R bestimmt werden. Alternativ kann die Bestimmung von Paw_ei während einer am Ende der Inspirationsphase stattfindenden Okklusion durchgeführt werden wie oben in Bezug zu Fig. 2 beschrieben. Anhand der gemessenen Werte von Paw_ei_n und Peso_ei_n wird dann ein neuer Wert für den maximalen

Druck am Ende der Inspirationsphase Paw_max_n+1 bestimmt. Die Bestimmung kann dabei nach folgender Beziehung erfolgen:

$$\text{Paw\_max\_n+1} = \ [(\text{Paw\_ei\_n} - \text{PEEP\_n})/(\text{Ptp\_ei\_n} - \text{Ptp\_ee\_n})]$$
$$* \text{Ptp\_ei\_max}$$

mit:

| | |
|---|---|
| Ptp_ei_max: | vorbestimmter maximaler transpulmonaler Druck am Ende einer Inspirationsphase, |
| Ptp_ei_n: | im aktuellen Atemzylus |
| | oder in m vorangehendenAtemzyklen bestimmter transpulmonaler Druck am Ende einer Inspirationsphase, |
| Ptp_ee_n: | im aktuellen Atemzyklus |
| | oder in m vorangehenden Atemzyklen bestimmter transpulmonaler Druck am Ende einer Expirationsphase, |
| Paw_ei_n | im aktuellen Atemzyklus |
| | oder in m vorangehenden Atemzyklen bestimmter Atemwegsdruck am Ende einer Inspirationsphase; |
| PEEP_n | im aktuellen Atemzyklus |
| | oder in m vorangehenden Atemzyklen bestimmter PEEP; |
| | mit m ≥ 1. |

Schritt 114 bestimmt erneut eine vorbestimmte Anzahl y von Atemzyklen Wartezeit, wobei gilt y ≥ 1, oder alternativ kann auch eine vorbestimmte Wartezeit, nach deren Ablauf der Prozess zurückspringt zur Bestimmung eines neuen PEEP in Schritt 108. Die vorangehend beschriebene Prozedur bestimmt sowohl den positiven endexpiratorischen Druck PEEP als auch den maximalen Atemwegsdruck jeweils patientenbezogen und situationsbezogen anhand der jeweils aktuell bestimmten Werte für den transpulmonalen Druck am Ende der Expirationsphase Ptp_ee und für den transpulmonalen Druck am Ende der Inspirationsphase Ptp_ei. Es sei darauf hingewiesen, dass in einfacheren Ausführungsbeispielen auch entsprechende Prozeduren denkbar sind, bei denen lediglich eine patientenbezogene und situationsbezogene Bestimmung des PEEP anhand des transpulmonalen Drucks am Ende der Expirationsphase Ptp_ee durchgeführt wird (und der maximale Atemwegsdruck gar nicht oder auf andere Weise bestimmt wird) oder bei denen lediglich eine patientenbezogene und situationsbezogene Bestimmung des maximalen Atemwegsdrucks anhand des transpulmonalen Drucks am Ende der Inspirationsphase Ptp_ei durchgeführt wird (und der PEEP gar nicht oder auf andere Weise festgelegt wird). Die entsprechenden Schritte der oben beschriebenen Prozedur entfallen dann.

**Patentansprüche**

1. System zur automatisierten Einstellung eines durch eine Beatmungseinrichtung (10) vorgegebenen Drucks , umfassend

   eine Druckermittlungsanordnung zum Ermitteln eines transpulmonalen Drucks am Ende einer Expirationsphase (Ptp_ee) und/oder eines transpulmonalen Drucks am Ende einer Inspirationsphase (Ptp_ei), **gekennzeichnet durch** eine Einrichtung zur automatisierten Einstellung des durch die Beatmungseinrichtung (10) vorgegebenen Drucks auf Grundlage des ermittelten transpulmonalen Drucks am Ende der Expirationsphase (Ptp_ee) und/oder des ermittelten transpulmonalen Drucks am Ende der Inspirationsphase (Ptp_ei).
   wobei das System dazu ausgebildet ist, einen durch die Beatmungseinrichtung vorgegebenen positiven endexpiratorischen Druck (PEEP) auf Grundlage des ermittelten transpulmonalen Drucks am Ende der Expirationsphase (Ptp_ee) automatisiert einzustellen; und/oder dazu ausgebildet ist, einen durch die Beatmungseinrichtung vorgegebenen maximalen Atemwegsdruck (Paw_max) auf Grundlage des ermittelten transpulmonalen Drucks am Ende der Inspirationsphase (Ptp_ei), des transpulmonalen Drucks am Ende der Expirationsphase (Ptp_ee) sowie des positiven endexpiratorischen Drucks (PEEP), automatisiert einzustellen;
   wobei das System derart ausgebildet ist, dass es den positiven endexpiratorischen Druck in einem nachfolgenden Atemzyklus (PEEP_n + 1) nach Maßgabe einer Abweichung des in m vorangehenden Atemzyklen ermittelten transpulmonalen Drucks am Ende einer Expirationsphase (Ptp_ee_n) von einem normierten transpulmonalen Druck am Ende einer Expirationsphase (Ptp_ee_ideal) bestimmt, und/oder dass es den maximalen Atemwegsdruck in einem nachfolgenden Atemzyklus (Paw_max_n+1) anhand einer Differenz zwischen einem in m vorangehenden Atemzyklen ermittelten Atemwegsdruck am Ende einer Inspirationsphase (Paw_ei_n) und einem in m vorangehenden Atemzyklen angewendeten positiven endexpiratorischen Druck (PEEP_n) bestimmt wobei m $\geq$ 1.

2. System nach Anspruch 1, wobei die Druckermittlungsanordnung eine zur Bestimmung eines alveolären Drucks (Palv) sowie eine zur Bestimmung eines oesophagealen Drucks (Peso) ausgelegte Sensorik aufweist, und den jeweiligen transpulmonalen Druck (Ptp) anhand einer Differenz zwischen dem jeweiligen alveolären Druck (Palv) und dem jeweiligem oesophagealen Druck (Peso) ermittelt.

3. System nach Anspruch 2, wobei die Sensorik zur Bestimmung des oesophagealen Drucks (Peso) einen zur Erfassung des oesophagealen Drucks (Peso) in den Oesophagus (34) einführbaren Katheter mit Ballonsonde (46) aufweist.

4. System nach Anspruch 2 oder 3, wobei die Sensorik zur Bestimmung des alveolären Drucks (Palv) derart ausgebildet ist, dass sie den Atemwegswiderstand (R) sowie den Gasfluss im Atemweg am Ende einer Expirationsphase (V_ee) und/oder den Gasfluss im Atemweg am Ende einer Inspirationsphase (V_ei) bestimmt und den jeweiligen alveolären Druck (Palv_ee; Palv_ei) anhand des jeweiligen Gasflusses (V_ee; V_ei) sowie des Atemwegswiderstands (R) bestimmt.

5. System nach einem der Ansprüche 2 bis 4, wobei die Sensorik zur Bestimmung des alveolären Drucks (Palv) einen am Tubusanfang angeordneten oder einem Atemwegeingangsventil (18) der Beatmungseinrichtung (10) zugeordneten Atemwegsdrucksensor zur Erfassung des eingangsseitigen Atemwegsdrucks (Paw) umfasst.

6. System nach Anspruch 5, wobei die Sensorik zur Bestimmung des alveolären Drucks (Palv) eine Anordnung zum Herstellen einer kurzzeitigen Okklusion des Atemwegs umfasst und derart ausgebildet ist, dass sie den alveolären Druck am Ende einer Expirationsphase (Palv_ee) und/oder den alveolären Druck am Ende einer Inspirationsphase (Palv-ei) anhand des während einer am Ende der Expirationsphase und/oder am Ende der Inspirationsphase stattfindenden Okklusion erfassten eingangsseitigen Atemwegsdrucks (Paw_ee; Paw_ei) bestimmt.

7. System nach Anspruch 6, wobei die Anordnung zum Herstellen einer kurzzeitigen Okklusion derart ausgebildet ist, dass sie einen gleichzeitigen Verschluss eines Atemwegeingangsventils (18) und eines Atemwegausgangsventils (24) der Beatmungseinrichtung (10) über einen vorbestimmten Okklusionszeitraum herbeiführt.

8. System nach einem der Ansprüche 1 bis 7, welches derart ausgebildet ist, dass es anhand von für einen jeweiligen Atemzyklus (n) ermittelten Werten den positiven endexpiratorischen Druck (PEEP_n+1) für den nachfolgenden Atemzyklus und/oder den maximalen Atemwegsdruck (Paw_max_n+1) für den nachfolgenden Atemzyklus ermitteltund insbesondere den positiven endexpiratorischen Druck für den nachfolgenden Atemzyklus (PEEP_n+1) und/oder den maximalen Atemwegsdruck (Paw_max_n+1) für den nachfolgenden Atemzyklus anhand von m vorangehenden Atemzyklen ermittelt.

9. System nach einem der Ansprüche 1 bis 8, welches derart ausgebildet ist, dass es den für den nachfolgenden Atemzyklus ermittelten positiven endexpiratorischen Druck (PEEP_n+1) mit dem für den aktuellen Atemzyklus eingestellten positiven endexpiratorischen Druck (PEEP_n) vergleicht und den positiven endexpiratorischen Druck nur dann verändert, wenn die Differenz zwischen beiden Werten einen vorbestimmten Schwellenwert übersteigt und/oder den für den nachfolgenden Atemzyklus ermittelten maximalen Atemwegsdruck (Paw_max_n+1) mit dem für den aktuellen Atemzyklus eingestellten maximalen Atemwegsdruck (Paw_max_n) vergleicht und den maximalen Atemwegsdruck nur dann verändert, wenn die Differenz zwischen beiden Werten einen vorbestimmten Schwellenwert übersteigt.

10. System nach Anspruch 8 oder 9, welches derart ausgebildet ist, dass es für einen jeweiligen Atemzyklus anhand des Gasflusses im Atemweg am Ende der Expirationsphase und/oder am Ende der Inspirationsphase (V_ee; V_ei) sowie anhand des Atemwegswiderstands (R) einen vorläufigen Wert für den positiven endexpiratorischen Atemwegsdruck im nachfolgenden Atemzyklus (PEEP_n+1_vorläufig) und/oder einen vorläufigen Wert für den maximalen Atemwegsdruck im nachfolgenden Atemzyklus (Paw_max_n+_vorläufig) bestimmt und dann, wenn der vorläufige Wert für den positiven endexpiratorischen Druck im nachfolgenden Atemzyklus (PEEP_n+1_vorläufig) sich vom dem eingestellten Wert für den positiven endexpiratorischen Druck (PEEP) um einen vorbestimmten Schwellenwert oder mehr unterscheidet und/oder der vorläufige Wert für den maximalen Atemwegsdruck im nachfolgenden Atemzyklus (Paw_max_n+1_vorläufig) sich von dem eingestellten Wert für den maximalen Atemwegsdruck (Paw_max) um einen vorbestimmten Schwellenwert oder mehr unterscheidet, einen neuen Wert für den positiven endexpiratorischen Druck im nachfolgenden Atemzyklus (PEEP_n+ 1) und/oder einen neuen Wert für den maximalen Atemwegsdruck im nachfolgenden Atemzyklus (Paw_max_n+1) nach Maßgabe einer kurzzeitigen Okklusion bestimmt.

11. System nach einem der Ansprüche 1 bis 10, welches derart ausgebildet ist, dass es den positiven endexpiratorischen Druck in dem nachfolgenden Atemzyklus (PEEP_n+1)gemäß folgender Formel bestimmt:

$$\text{PEEP\_n+1 = PEEP\_n + f * (Ptp\_ee\_ideal – Ptp\_ee\_n)}$$

mit:

PEEP_n: positiver endexpiratorischer Druck im aktuellen Atemzyklus oder in m vorangehenden Atemzyklen
Ptp_ee_ideal: normierter transpulmonaler Druck am Ende einer Expirationsphase,
Ptp_ee_n: im aktuellen Atemzyklus oder in m vorangehenden Atemzyklen bestimmter transpulmonaler Druck am Ende einer Expirationsphase, mit $m \geq 1$,
$0 \leq f \leq 1$, insbesondere $0.01 \leq f \leq 0.8$, insbesondere $0.05 \leq f \leq 0.5$, insbesondere $f = 0.2$.

12. System nach Anspruch 11, wobei der normierte transpulmonale Druck am Ende einer Expirationsphase (Ptp_ee_ideal) anhand einer vorbestimmten Beziehung zwischen dem normierten transpulmonalen Druck am Ende einer Expirationsphase (Ptp_ee_ideal) und dem Sauerstoffanteil des Atemgases (FiO2) ermittelbar ist und/oder ein Startwert für den positiven endexpiratorischen Druck anhand einer vorbestimmten Beziehung zwischen dem positivem endexpiratorischem Druck (PEEP) und dem Sauerstoffanteil des Atemgases (FiO2) ermittelbar ist, wobei die vorbestimmte Beziehung ggf. davon abhängig ist, ob der Sauerstoffanteil des Atemgases (FiO2) gegenüber dem seither eingestellten Sauerstoffanteil erhöht oder verringert wird.

13. System nach einem der Ansprüche 1 bis 12, welches derart ausgebildet ist, dass es den maximalen Atemwegsdruck in dem nachfolgenden Atemzyklus (Paw_max_n+1) nach folgender Formel bestimmt:

$$\text{Paw\_max\_n+1 = [(Paw\_ei\_n – PEEP\_n)/(Ptp\_ei\_n – Ptp\_ee\_n)]}$$
$$\text{* Ptp\_ei\_max}$$

mit:

Ptp_ei_max: vorbestimmter maximaler transpulmonaler Druck am Ende einer Inspirationsphase,

Ptp_ei_n: im aktuellen Atemzyklus oder in m vorangehenden Atemzyklen bestimmter transpulmonaler Druck am Ende einer Inspirationsphase,

Ptp_ee_n: im aktuellen Atemzyklus oder in m vorangehenden Atemzyklen bestimmter transpulmonaler Druck am Ende einer Expirationsphase,

Paw_ei_n: im aktuellen Atemzyklus oder in m vorangehenden Atemzyklen bestimmter Atemwegsdruck am Ende einer Inspirationsphase,

PEEP n im aktuellen Atemzyklus oder in m vorangehenden Atemzyklen bestimmter PEEP.

14. System nach Anspruch 13, wobei der vorbestimmte maximale transpulmonale Druck am Ende einer Inspirationsphase (Ptp_ei_max) zwischen 15 und 20 cm $H_2O$ liegt und/oderein Startwert für den maximalen Atemwegsdruck am Ende einer Inspirationsphase (Paw_ei_max) bei 30 cm $H_2O$ liegt.

15. Beatmungseinrichtung, umfassend ein System zur automatischen Einstellung eines positiven endexpiratorischen Drucks nach einem der vorangehenden Ansprüche.

**Claims**

1. A system for automated adjustment of a pressure set by a ventilation device (10), comprising
a pressure detection arrangement for detecting a transpulmonary pressure at the end of an expiration phase (Ptp_ee) and/or a transpulmonary pres - sure at the end of an inspiration phase (Ptp ei),
   **characterised by**
   a means for automated adjustment of the pressure set by the ventilation de - vice (10) on the basis of the transpulmonary pressure detected at the end of the expiration phase (Ptp_ee) and/or the transpulmonary pressure detected at the end of the inspiration phase (Ptp_ei),
   wherein the system is configured to automatically adjust a positive end-expi - ratory pressure (PEEP) set by the ventilation device on the basis of the transpulmonary pressure detected at the end of the expiration phase (PTP_ee); and/ or configured to automatically adjust a maximum airway pressure (Paw_max) set by the ventilation device on the basis of the transpulmonary pressure detected at the end of the inspiration phase (Ptp_ei), the transpulmonary pressure at the end of the expiration phase (Ptp_ee) and the positive end-expiratory pressure (PEEP);
   wherein the system is designed to determine the positive end-expiratory pressure in a subsequent breathing cycle (PEEP_n+1) in accordance with a deviation of the transpulmonary pressure at the end of an expiration phase (Ptp_ee_n) detected in m preceding breathing cycles from a normalized transpulmonary pressure at the end of an expiration phase (Ptp_ee_ideal), and/or to determine the maximum airway pressure in a subsequent breath - ing cycle (Paw_max_n+1) on the basis of a difference between an airway pressure at the end of an inspiration phase (Paw_ei_n) detected in m pre - ceding breathing cycles and a positive end-expiratory pressure (PEEP_n) applied in m preceding breathing cycles,
   with m $\geq$ 1.

2. The system of claim 1,
wherein the pressure detection arrangement comprises a sensor means de - signed for determining an alveolar pressure (Palv) and for determining an esophageal pressure (Peso), and determines the respective transpulmonary pressure (Ptp) on the basis of a difference between the respective alveolar pressure (Palv) and the respective esophageal pressure (Peso).

3. The system of claim 2,
wherein the sensor means for determining the esophageal pressure (Peso) comprises a catheter with balloon probe (46) that is adapted to be inserted into the esophagus (34) for detecting the esophageal pressure (Peso).

4. The system of claim 2 or 3,
wherein the sensor means for determining the alveolar pressure (Palv) is formed such that it determines the airway resistance (R) and the gas flow in the airway at the end of an expiration phase (V_ee) and/or the gas flow in the airway at the end of an inspiration phase (V_ei) and determines the re - spective alveolar pressure (Palv_ee; Palv_ei) by way of the respective gas flow (V_ei; V_ee) and the airway resistance (R).

**5.** The system of any of claims 2 to 4,
wherein the sensor means for determining the alveolar pressure (Palv) com - prises an airway pressure sensor for determining the inlet-side airway pres - sure (Paw), arranged at the tube beginning or associated with an airway in - let valve (18) of the ventilation device (10).

**6.** The system of claim 5,
wherein the sensor means for determining the alveolar pressure (Palv) com - prises an arrangement for producing a short-time occlusion of the airway and is configured such that it determines the alveolar pressure at the end of an expiration phase (Palv_ee) and/or the alveolar pressure at end of an in - spiration phase (Palv_ei) by way of the inlet-side airway pressure (Paw_ee; Paw_ei) detected during an occlusion effected at the end of the expiration phase and/or at the end of the inspiration phase.

**7.** The system of claim 6,
wherein the arrangement for producing a short-time occlusion is formed such that it causes simultaneous closure of an airway inlet valve (18) and an airway outlet valve (24) of the ventilation device (10) over a predetermined occlusion period.

**8.** The system of any of claims 1 to 7,
which is designed to determine, on the basis of (n) values determined for a respective breathing cycle, the positive end-expiratory pressure (PEEP_n+1) for the subsequent breathing cycle and/or the maximum airway pressure (Paw_max_n+1) for the subsequent breathing cycle, and in partic - ular to determine the positive end-expiratory pressure for the subsequent breathing cycle (PEEP_n+1) and/or the maximum airway pressure (Paw_max_n+1) for the subsequent breathing cycle on the basis of m pre - ceding breathing cycles.

**9.** The system of any of claims 1 to 8,
which is designed to compare the positive end-expiratory pressure (PEEP_n+1) determined for the subsequent breathing cycle with the posi - tive end-expiratory pressure (PEEP_n) set for the current breathing cycle, and to change the positive end-expiratory pressure only if the difference be - tween the two values exceeds a predetermined threshold value, and/or to compare the maximum airway pressure (Paw_max_n+1) determined for the subsequent breathing cycle with the maximum airway pressure (Paw_max_n) set for the current breathing cycle, and to change the maxi - mum airway pressure only if the difference between the two values exceeds a predetermined threshold value.

**10.** The system of claim 8 or 9,
which is designed to determine, for a respective breathing cycle, a preliminary value for the positive end-expiratory airway pressure in the subsequent breathing cycle (PEEP_n+1_preliminary) and/or a preliminary value for the maximum airway pressure in the subsequent breathing cycle (Paw_max_n+1_preliminary) on the basis of the gas flow in the airway at the end of the expiration phase and/or at the end of the inspiration phase (V_ee; V_ei) and on the basis of the airway resistance (R) and, if the preliminary value for the positive end-expiratory pressure in the subsequent breathing cycle (PEEP_n+1_preliminary) differs from the value set for the positive end-expiratory pres - sure (PEEP) by a predetermined threshold value or more and/or the preliminary value for the maximum airway pressure in the subsequent breathing cycle (Paw_max_n+1_preliminary) differs from the value set for the maximum airway pressure (Paw_max) by a predetermined threshold value or more, to determine a new value for the positive end-expiratory pressure in the subsequent breathing cycle (PEEP_n+1) and/or a new value for the maximum airway pressure in the subsequent breathing cycle (Paw_max_n+1) in accordance with a short-time occlusion.

**11.** The system of any of claims 1 to 10,
which is designed to determine the positive end-expiratory pressure in the subsequent breathing cycle (PEEP_n+1) according to the following formula:

$$PEEP\_n+1 = PEEP\_n + f * (Ptp\_ee\_ideal - Ptp\_ee\_n)$$

with:

   PEEP_n: positive end-expiratory pressure in the current breathing cycle or in m preceding breathing cycles,
   Ptp_ee_ideal: normalized transpulmonary pressure at the end of an expiration phase,

Ptp_ee_n: transpulmonary pressure at the end of an expiration phase determined in the current breathing cycle or in m preceding breathing cycles, with m ≥ 1,
$0 \leq f \leq 1$, in particular $0.01 \leq f \leq 0.8$, in particular $0.05 \leq f \leq 0.5$, in particular, f = 0.2.

12. The system of claim 11,
wherein the normalized transpulmonary pressure at the end of an expiration phase (Ptp_ee_ideal) can be determined on the basis of a predetermined relationship between the normalized transpulmonary pressure at the end of an expiration phase (Ptp_ee_ideal) and the oxygen content of the breathing gas (FiO2), and a starting value for the positive end-expiratory pressure can be determined on the basis of a predetermined relationship between the positive end-expiratory pressure (PEEP) and the oxygen content of the breathing gas (FiO2), the predetermined relationship being dependent on whether the oxygen content of the breathing gas (FiO2) is increased or de - creased in relation to the oxygen content set before.

13. The system of any of claims 1 to 12,
which is designed to determine the maximum airway pressure in the subse - quent breathing cycle (Paw_max_n+1) according to the following formula:

$$\text{Paw\_max\_n+1} = [(\text{Paw\_ei\_n} - \text{PEEP\_n})/(\text{Ptp\_ei\_n} - \text{Ptp\_ee\_n})] * \text{Ptp\_ei\_max}$$

with:

Ptp_ei_max: predetermined maximum transpulmonary pressure at the end of an inspiration phase,
Ptp_ei_n: transpulmonary pressure at the end of an inspiration phase determined in the current breathing cycle or in m preceding breathing cycles,
Ptp_ee_n: transpulmonary pressure at the end of an expiration phase determined in the current breathing cycle or in m preceding breathing cycles,
Paw_ei_n: airway pressure at the end of an inspiration phase determined in the current breathing cycle or in m preceding breathing cycles,
PEEP_n: PEEP determined in the current breathing cycle or in m preceding breathing cycles.

14. The system of claim 13,
wherein the predetermined maximum transpulmonary pressure at the end of an inspiration phase (Ptp_ei_max) is between 15 and 20 cm $H_2O$ and/or a starting value for the maximum airway pressure at the end of an inspiration phase (Paw_ei_max) is about 30 cm $H_2O$.

15. A ventilation device comprising a system for automated adjustment of a positive end-expiratory pressure in accordance with any of the preceding claims.

**Revendications**

1. Système destiné au réglage automatisé d'une pression prédéfinie par l'intermédiaire d'un mécanisme d'assistance respiratoire (10), comprenant :

un agencement de détermination de la pression, destiné à la détermination d'une pression transpulmonaire à la fin d'une phase d'expiration (Ptp_ee) et/ou d'une pression transpulmonaire à la fin d'une phase d'inspiration (Ptp_ei) ;
**caractérisé par**
un mécanisme destiné au réglage automatisé de la pression prédéfinie par l'intermédiaire du mécanisme d'assistance respiratoire (10), sur la base de la pression transpulmonaire déterminée à la fin de la phase d'expiration (Ptp_ee) et/ou de la pression transpulmonaire déterminée à la fin de la phase d'inspiration (Ptp_ei) ;
dans lequel le système est réalisé dans le but de régler de manière automatisée une pression positive en fin d'expiration (PEEP), prédéfinie par l'intermédiaire du mécanisme d'assistance respiratoire, sur la base de la pression transpulmonaire déterminée à la fin de la phase d'expiration (Ptp_ee) ; et/ou est réalisé dans le but de régler de manière automatisée une pression maximale des voies aériennes (Paw_max) prédéfinie par l'intermédiaire du mécanisme d'assistance respiratoire sur la base de la pression transpulmonaire déterminée à la fin de la phase d'inspiration (Ptp_ei), de la pression transpulmonaire à la fin de la phase d'expiration (Ptp_ee) et sur la base de la pression positive en fin d'expiration (PEEP) ;

dans lequel le système est réalisé d'une manière telle qu'il détermine la pression positive en fin d'expiration dans un cycle respiratoire ultérieur (PEEP_n+1) en conformité avec une divergence entre la pression transpulmonaire à la fin d'une phase d'expiration (Ptp_ee_n), déterminée au cours de m cycles respiratoires précédents et une pression transpulmonaire normalisée à la fin d'une phase d'expiration (Ptp_ee_ideal) ; et/ou d'une manière telle qu'il détermine la pression maximale des voies aériennes dans un cycle respiratoire ultérieur (Paw_max_n+1) en conformité avec une différence entre une pression des voies aériennes à la fin d'une phase d'inspiration (Paw_ei_n), déterminée au cours de m cycles respiratoires précédents et une pression positive en fin d'expiration (PEEP_n) appliquée au cours de m cycles respiratoires précédents ;

dans lequel m est égal ou supérieur à 1.

2. Système selon la revendication 1, dans lequel l'agencement de détermination de la pression présente un système de détection conçu pour la détermination d'une pression alvéolaire (Palv) ainsi qu'un système de détection conçu pour la détermination d'une pression œsophagienne (Peso), et détermine la pression transpulmonaire respective (Ptp) en conformité avec une différence entre la pression alvéolaire respective (Palv) et la pression œsophagienne respective (Peso).

3. Système selon la revendication 2, dans lequel le système de détection présente, pour la détermination de la pression œsophagienne (Peso), un cathéter du type possédant une sonde à ballonnet (46) qui peut être introduit dans l'œsophage (34) dans le but d'enregistrer la pression œsophagienne (Peso).

4. Système selon la revendication 2 ou 3, dans lequel le système de détection destiné à la détermination de la pression alvéolaire (Palv) est réalisé d'une manière telle qu'il détermine la résistance des voies aériennes (R) ainsi que le flux de gaz dans les voies aériennes à la fin d'une phase d'expiration (V_ee) et/ou le flux de gaz dans les voies aériennes à la fin d'une phase d'inspiration (V_ei) et d'une manière telle qu'il détermine la pression alvéolaire respective (Palv_ee ; Palv_ei) sur la base du flux de gaz respectif (V_ee ; V_ei) et sur la base de la résistance des voies aériennes (R).

5. Système selon l'une quelconque des revendications 2 à 4, dans lequel le système de détection destiné à la détermination de la pression alvéolaire (Palv) comprend un capteur de la pression des voies aériennes disposé au début de la sonde d'intubation ou attribué à une valve d'entrée des voies aériennes (18) du mécanisme d'assistance respiratoire (10), pour l'enregistrement de la pression des voies aériennes (Paw) du côté de l'entrée.

6. Système selon la revendication 5, dans lequel le système de détection destiné à la détermination de la pression alvéolaire (Palv) comprend un agencement destiné à la mise en place d'une occlusion des voies aériennes de courte durée et est réalisé d'une manière telle qu'il détermine la pression alvéolaire à la fin d'une phase d'expiration (Palv_ee) et/ou la pression alvéolaire à la fin d'une phase d'inspiration (Palv_ei) en conformité avec la pression des voies aériennes (Paw_ee ; Paw_ei) du côté de l'entrée, enregistrée au cours d'une occlusion qui se déroule à la fin de la phase d'expiration et/ou à la fin de la phase d'inspiration.

7. Système selon la revendication 6, dans lequel l'agencement destiné à la mise en place d'une occlusion des voies aériennes de courte durée est réalisé d'une manière telle qu'il induit une fermeture simultanée d'une valve d'entrée (18) des voies aériennes et d'une valve de sortie (24) des voies aériennes du mécanisme d'assistance respiratoire (10) au cours d'un laps de temps d'occlusion prédéterminé.

8. Système selon l'une quelconque des revendications 1 à 7, qui est réalisé d'une manière telle qu'il détermine, en conformité avec des valeurs déterminées pour un cycle respiratoire respectif (n), la pression positive en fin d'expiration (PEEP_n+1) pour le cycle respiratoire suivant et/ou la pression maximale des voies aériennes (Paw_max_n+1) pour le cycle respiratoire suivant et qu'il détermine en particulier la pression positive en fin d'expiration pour le cycle respiratoire suivant (PEEP_n+1) et/ou la pression maximale des voies aériennes (Paw_max_n+1) pour le cycle respiratoire suivant en se référant à m cycles respiratoires précédents.

9. Système selon l'une quelconque des revendications 1 à 8, qui est réalisé d'une manière telle qu'il compare la pression positive en fin d'expiration (PEEP_n+1) déterminée pour le cycle respiratoire suivant à la pression positive en fin d'expiration (PEEP_n) réglée pour le cycle respiratoire en cours et qu'il modifie ensuite la pression positive en fin d'expiration uniquement lorsque la différence entre les deux valeurs dépasse vers le haut une valeur seuil prédéfinie et/ou d'une manière telle qu'il compare la pression maximale des voies aériennes (Paw_max_n+1) déterminée pour le cycle respiratoire suivant à la pression maximale des voies aériennes (Paw_max_n) réglée pour le cycle respiratoire en cours et qu'il modifie ensuite la pression maximale des voies aériennes uniquement lorsque la différence entre les deux valeurs dépasse vers le haut une valeur seuil prédéfinie.

10. Système selon la revendication 8 ou 9, qui est réalisé d'une manière telle qu'il détermine, pour un cycle respiratoire respectif, en conformité avec le flux de gaz dans les voies aériennes à la fin de la phase d'expiration et/ou à la fin de la phase d'inspiration (V_ee ; V_ei) et également en conformité avec la résistance des voies aériennes (R), une valeur temporaire pour la pression positive en fin d'expiration dans le cycle respiratoire suivant (PEEP_n+1_vorläufig) et/ou une valeur temporaire pour la pression maximale des voies aériennes dans le cycle respiratoire suivant (Paw_max_n+1_vorläufig) et, lorsque la valeur temporaire pour la pression positive en fin d'expiration dans le cycle respiratoire suivant (PEEP_n+1_vorläufig) se différencie de la valeur réglée pour la pression positive en fin d'expiration (PEEP) à concurrence d'une valeur seuil prédéfinie ou plus et/ou lorsque la valeur temporaire pour la pression maximale des voies aériennes dans le cycle respiratoire suivant (Paw_max_n+1_vorläufig) se différencie de la valeur réglée pour la pression maximale des voies aériennes (Paw_max) à concurrence d'une valeur seuil prédéfinie ou plus, détermine une nouvelle valeur pour la pression positive en fin d'expiration dans le cycle respiratoire suivant (PEEP_n+1) et/ou une nouvelle valeur pour la pression maximale des voies aériennes dans le cycle respiratoire suivant (Paw_max_n+1) en conformité avec une occlusion de courte durée.

11. Système selon l'une quelconque des revendications 1 à 10, qui est réalisé d'une manière telle qu'il détermine la pression positive en fin d'expiration dans le cycle respiratoire suivant (PEEP_n+1) en se référant à la formule suivante :

$$PEEP\_n+1 = PEEP\_n + f * (Ptp\_ee\_ideal - Ptp\_ee\_n)$$

dans laquelle :

PEEP_n : la pression positive en fin d'expiration dans le cycle respiratoire en cours ou dans m cycles respiratoires précédents ;
Ptp_ee_ideal : la pression transpulmonaire normalisée à la fin d'une phase d'expiration ;
Ptp_ee_n : la pression transpulmonaire à la fin d'une phase d'expiration, déterminée dans le cycle respiratoire en cours ou dans m cycles respiratoires précédents, m étant supérieur ou égal à 1 ;

$0 \leq f \leq 1$, en particulier $0,01 \leq f \leq 0,8$, en particulier $0,05 \leq f \leq 0,5$, en particulier $f = 0,2$.

12. Système selon la revendication 11, dans lequel on peut déterminer la pression transpulmonaire normalisée à la fin d'une phase d'expiration (Ptp_ee_ideal) en se basant sur une relation prédéfinie entre la pression transpulmonaire normalisée à la fin d'une phase d'expiration (Ptp_ee_ideal) et la fraction d'oxygène du gaz respiratoire (FiO2) et/ou on peut déterminer une valeur de départ pour la pression positive en fin d'expiration sur base d'une relation prédéfinie entre la pression positive en fin d'expiration (PEEP) et la fraction d'oxygène du gaz respiratoire (FiO2) ; dans lequel la relation prédéfinie dépend, le cas échéant, du fait de savoir si la fraction d'oxygène du gaz respiratoire (FiO2) est augmentée ou est diminuée par rapport à la fraction d'oxygène réglée depuis lors.

13. Système selon l'une quelconque des revendications 1 à 12, qui est réalisé d'une manière telle qu'il détermine la pression maximale des voies aériennes dans le cycle respiratoire suivant (Paw_max_n+1) en se référant à la formule suivante :

$$Paw\_max\_n+1 \; : \; [(Paw\_ei\_n - PEEP\_n)/(Ptp\_ei\_n - Ptp\_ee\_n)] \; * \; Ptp\_ei\_max$$

dans laquelle :

Ptp_ei_max : la pression transpulmonaire maximale prédéfinie à la fin d'une phase d'inspiration ;
Ptp_ei_n : la pression transpulmonaire à la fin d'une phase d'inspiration déterminée dans le cycle respiratoire en cours ou dans m cycles respiratoires précédents ;

Ptp_ee_n : la pression transpulmonaire à la fin d'une phase d'expiration déterminée dans le cycle respiratoire en cours ou dans m cycles respiratoires précédents ;

Paw_ei_n : la pression des voies aériennes à la fin d'une phase d'inspiration déterminée dans le cycle respiratoire en cours ou dans m cycles respiratoires précédents ;

PEEP_n : la PEEP déterminée dans le cycle respiratoire en cours ou dans m cycles respiratoires précédents.

14. Système selon la revendication 13, dans lequel la pression transpulmonaire maximale prédéterminée à la fin d'une phase d'inspiration (Ptp_ei_max) se situe entre 15 et 20 cm de $H_2O$ et/ou une valeur de départ pour la pression maximale des voies aériennes à la fin d'une phase d'inspiration (Paw_ei_max) s'élève à 30 cm de $H_2O$.

15. Mécanisme d'assistance respiratoire comprenant un système destiné au réglage automatique d'une pression positive en fin d'expiration selon l'une quelconque des revendications précédentes.

Fig. 2

Fig. 3

Fig.4a

Fig.4b

Fig.5

| FiO2 <= | 0.5 | 0.6 | 0.7 | 0.8 | 0.9 | 1.0 |
|---|---|---|---|---|---|---|
| Ptp_ee_ideal, mbar | 0 | 2 | 4 | 6 | 8 | 10 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2091429 B1

**In der Beschreibung aufgeführte Nicht-Patentliteratur**

- The Acute Respiratory Distress Syndrome Network. *The New England Journal of Medicine,* 2000, vol. 342, 1301-1308
- **GRASSO et al.** *American Journal of Respiratory Critical Care,* 2007, vol. 176, 761-767
- **BROCHARD L.** *Critical Care,* 2006, vol. 10, 156-158
- Mechanical Ventilation Guided by Esophageal Pressure in Acute Lung Injury. **TALMOR DANIEL et al.** NEW ENGLAND JOURNAL OF MEDICINE, THE - NEJM. MASSACHUSETTS MEDICAL SOCIETY, 13. November 2008, vol. 359, 2095-2104
- **TALMOR D. et al.** *New England Journal of Medicine,* 2008, vol. 359, 2095-2104
- **BENDITT J.** *Resp. Care,* 2005, vol. 50, 68-77
- **LOTTI I.A. et al.** *Intensive Care Med.,* 1995, vol. 21, 406-413